# EUROPEAN PATENT APPLICATION

(11) **EP 2 412 302 A1**
(43) Date of publication of application: **01.02.2012**
(21) Application number: 10171266.9
(22) Date of filing: 29.07.2010
(51) Int. Cl.: A61B 1/31

(54) **Proctoscope**

(71) Applicant: Parburch Medical Developments Limited, Ipswich Suffolk IP1 9ET (GB)
(72) Inventor: Parker, Robert, Ipswich, suffolk IP1 9ET (GB)
(74) Representative: Gemmell, Peter Alan

(57) **Abstract**

A proctoscope (2) comprises a tubular proctoscope body (8) having a bore (10) and a handle (9) integrally formed with the proctoscope body; wherein
the proctoscope body (8) has a distal end (12) with an opening, for insertion into the anus of a patient, and a mounting member (30) for supporting a light source (6) for directing light down the bore (10) towards the distal end (12);
characterised in that a translucent window (54) is provided, which is integral with the proctoscope body (8) and provides a fluid tight seal between the bore (10) and the light source (6) such that, in use, a light source (6) supported in the mounting member (30) is shielded from foreign matter passing along the bore of the proctoscope body.

## Description

### BACKGROUND

### a. Field of the Invention

This invention relates to the field of proctoscopes and in particular, but not exclusively, to the field of disposable proctoscopes.

### b. Related Art

Proctoscopy is a common medical procedure in which the rectal lining of a patient is examined for signs of disease. The instrument used to perform this examination is a proctoscope, which comprises a straight, rigid tube about 10 cm in length with a removable obturator.

During proctoscopy, the proctoscope is lubricated and inserted into the anus and rectum of the patient and the obturator, which is present to ease insertion of the proctoscope, is removed to allow viewing of the rectal lining.

Traditionally, proctoscopes were made of metal and were designed to be used more than once, being sterilised between patients. To permit better inspection of the rectal lining, the proctoscope would be fitted with a light either at the distal end of the tube or within a channel provided along the length of the tube. This light would then be sterilized along with the proctoscope.

Disposable proctoscopes have been available for many years. These are generally made from a rigid, clear plastic material, and are typically injection-moulded. This design has a number of advantages including the fact that the clear plastic allows a better view of the rectal lining along the entire length of the proctoscope. Additionally, the disposable nature of the proctoscopes means that sterilization of the equipment between patients is no longer required. In many instances it is still desirable to include a light source in the proctoscope and this often consists of a fibre optic light source mounted along the side of the tube of the proctoscope that directs light down the tube towards the distal end. This light source is not disposable and therefore is designed to be easily inserted into and removed from the proctoscope.

However, there is a risk that foreign matter will pass along the tube of the proctoscope and contaminate the end of the fibre optic or other light source. This means that either the light source has to be sterilized between uses or that a new light source has to be used with each patient, which would be expensive.

### SUMMARY OF THE INVENTION

According to the invention there is provided a proctoscope as specified in claim 1. Preferred features are specified in the dependent claims.

### BRIEF DESCRIPTION OF THE DRAWINGS

The invention will now be further described, by way of example only, and with reference to the accompanying drawings, in which:
Figure 1 is a perspective view of a prior art disposable proctoscope assembly;
Figure 2 is a perspective view of the proctoscope of Figure 1;
Figure 3 is a perspective view of a proctoscope in accordance with an embodiment of the invention;
Figure 4 is a sectional view of the proctoscope of Figure 3;
Figures 5 and 6 are perspective views of a proctoscope in accordance with an embodiment of the invention, without and with a light source respectively.

### DETAILED DESCRIPTION

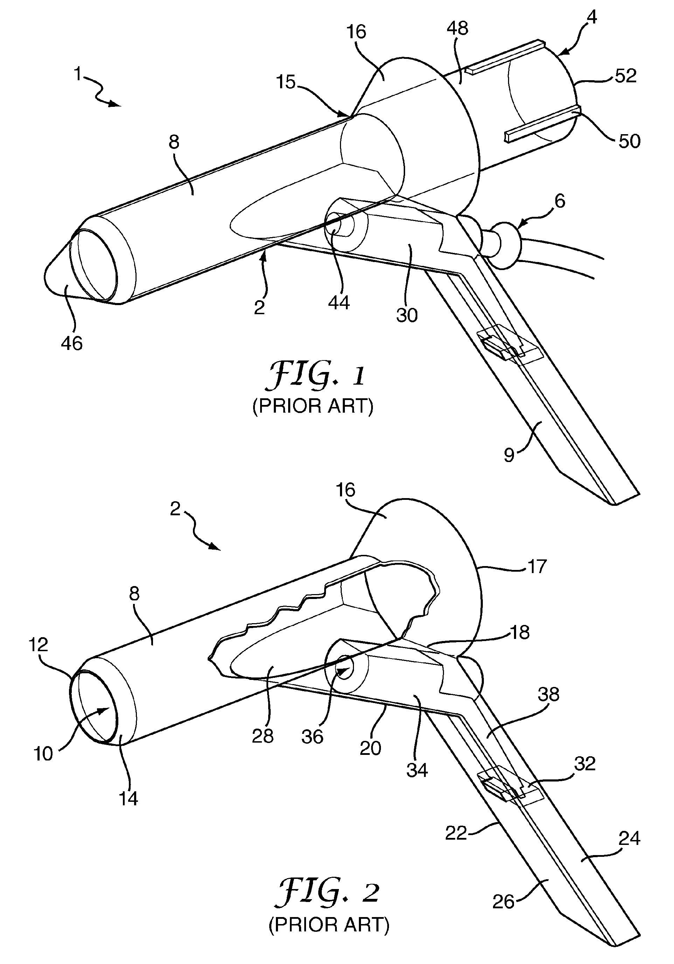

Figure 1 shows a typical proctoscope assembly 1. The proctoscope assembly 1 includes a proctoscope 2, an obturator 4 and a light source 6.

The proctoscope assembly may come in a range of sizes to suit different patients, for example the effective length of the assembly 1 may range from 40 to 70 mm, and the external diameter of the proctoscope 2 may range from 15 to 25 mm. In this case the smaller sizes would be suitable for paediatric use, while the larger sizes would be suitable for adult patients.

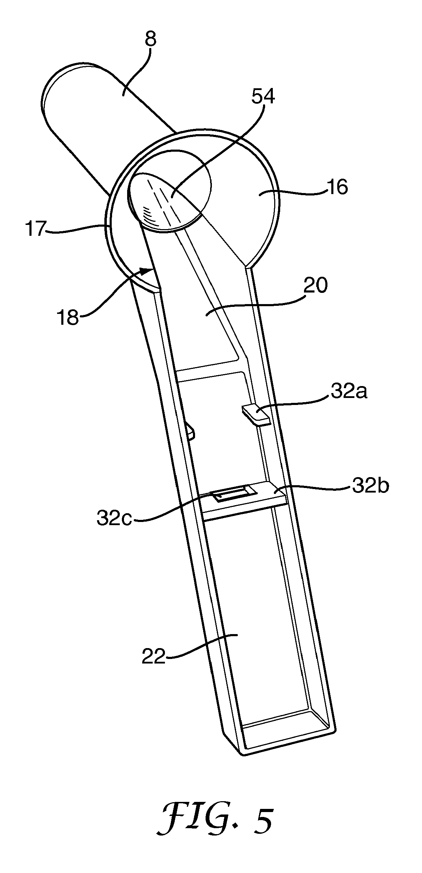

The proctoscope 2 comprises a generally tubular proctoscope body 8, in this example cylindrical, and a handle 9. As shown in the prior art example in Figures 1 and 2, the proctoscope body 8 has a cylindrical bore 10 coaxial with the longitudinal axis of the proctoscope body 8. Near the distal end 12 of the body 8 there is a tapered region 14. In this region 14 the diameter of the bore 10 remains constant and the external diameter of the proctoscope body 8 decreases to a minimum at the distal end 12 of the body 8. This tapering aids insertion of the proctoscope 2 into the anus of a patient. In a proximal portion 15 of the proctoscope body 8, a flange 16 extends generally outwardly from the body 8. The flange 16 acts to limit the extent to which the proctoscope 2 is inserted into a patient and makes it easier to view along the length of the proctoscope body 8. The flange 16 does not extend completely around the circumference of the tubular body 8 of the proctoscope 2 but terminates at an upper edge 18 of the handle 9 of the proctoscope 2, most clearly seen in Figure 5.

In this example, the handle 9 of the proctoscope 2 includes a connecting portion 20 and a grip portion 22. The handle 9 comprises two walls 24 substantially parallel to the longitudinal axis of the proctoscope body 8 and a front face 26. The upper edge 18 of the handle 9 also forms the upper edge of the connecting portion 20 and extends longitudinally from the rear edge 17 of the flange 16 along approximately half the length of the proctoscope body 8. The walls 24 and face 26 of the handle 9 are integrally formed with the proctoscope body 8 such that an elongate slot 28 is formed in the proctoscope body 8, the slot 28 being open at the rear edge 17 of the flange 16.

The connecting portion 20 extends radially outward and rearward from the proctoscope body 8 such that the front face 26 of the handle 9 in the connecting portion 20 is at an angle of less than 45° to the longitudinal axis of the body 8. The grip portion 22 extends radially outward from the proctoscope body 8 at the rear of the connecting portion 20. The front face 26 of the handle 9 in the grip portion 22 is at an angle of greater than 45° to the longitudinal axis of the body 8. In this way, there is a change in angle of the face 26 of the handle 9, relative to the longitudinal axis of the body 8, where the grip portion 22 joins the connecting portion 20. The grip portion 22 of the handle 9 does not have a rear face so that the entire rear of the proctoscope 2 is open, defined by the rear edge 17 of the flange 16 and the walls 24 of the handle 9.

A mounting member is provided to support the light source 6 in the proctoscope 2. In this prior art example, the mounting member is a guide block 30, shaped so as to fit within the handle 9 of the proctoscope 2 and held in place by retaining means 32. In this example, the retaining means 32 includes projections 32a on the inside faces of the side walls 24 of grip portion 22 of the handle 9 proximate the connecting portion 20, and a brace 32b extending between the side walls 24 of the grip portion 22 which includes a cut out 32c adjacent the front face 26 of the handle 9, seen most clearly in Figure 5. An upper section 34 of the guide block 30 is shaped to fit within the connecting portion 20 of the handle 9 and includes a cylindrical hole 36. The longitudinal axis of the hole 36 lies approximately parallel to the front face 26 of the handle 9 in the connecting portion 20 and defines a longitudinal axis of the upper section 34 of the guide block 30. A lower section 38 of the guide block 30 extends at an angle to the longitudinal axis of the upper section 34 and is shaped to fit within the grip portion 22 of the handle 9 bounded by the side walls 24 and the brace 32b. The lower section 38 of the guide block 30 includes two notches 42 that locate around the projections 32a, and a projection 40 that engages with the cut out 32c in the brace 32b to retain the guide block 30 in the correct position in the handle 9.

The guide block 30 is used to support the light source 6. It will be appreciated that in other embodiments, the light source 6 may be supported by other suitable mounting means for example clips or projections in the handle 9.

In use, the end 44 of a fibre optic light source 6 is pushed through the hole 36 in the guide block 30 so that the end 44 projects a short distance from the front of the guide block 30 but does not project into the bore 10 of the proctoscope body 8. The angle of the light source 6 when held within the guide block 30 is such that light is directed at an angle down the length of the bore 10 towards the distal end 12 of the proctoscope body 8.

With further reference to Figure 1, the obturator 4 is a cylindrical tube with an outside diameter approximately the same as the diameter of the bore 10 of the proctoscope body 8, such that there is a tight fit when the obturator 4 is inserted in the proctoscope body 8. At one end of the obturator 4 there is a tapering tip 46 with a closed rounded end, which, when the obturator 4 is inserted in the proctoscope 2, extends beyond the distal end 12 of the proctoscope body 8. At a distance from the tip 46, corresponding to the distance between the distal end 12 of the proctoscope body 8 and the flange 16, the diameter of the obturator 4 increases such that a rear section 48 of the obturator 4 is formed by a length of tube with a diameter greater than the diameter of a front section of the obturator 4. This increase in diameter serves to prevent the obturator 4 being pushed too far into the proctoscope body 8 and also makes it easier to grip the rear section 48 of the obturator. To improve grip further, ridges 50 running parallel to the longitudinal axis of the obturator 4 are provided proximate the rear edge 52.

In use, the obturator 4 is positioned in the proctoscope body 8, as shown in Figure 1, and a fibre optic light source 6 is pushed into the guide block 30. The proctoscope assembly 1 is then inserted into the anus of a patient. Once in position, the obturator 4 is removed from the proctoscope 2 to allow the rectal lining of the patient to be examined. During the examination, light from the light source 6 is directed along the bore 10 of the proctoscope body 8.

Once the obturator 4 has been removed from the proctoscope 2 there is a continuous and open channel from the distal end 12 of the proctoscope body 8 to the end 44 of the light source 6 due to the slot 28 in the proctoscope body 8. This has the disadvantage that any foreign matter passing along the bore 10 of the proctoscope 2 may contaminate the end 44 of the light source 6.

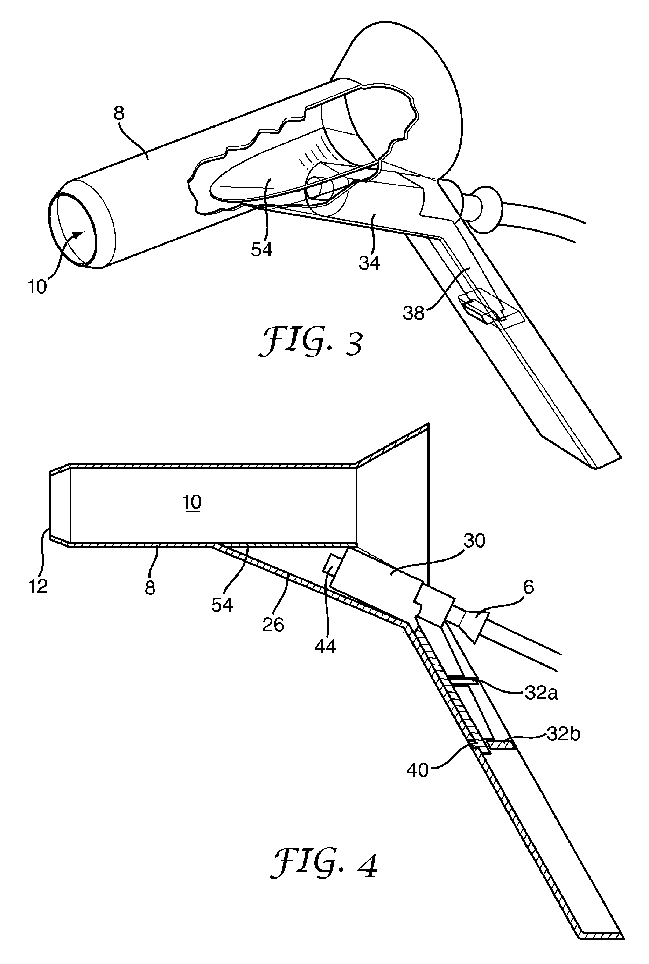

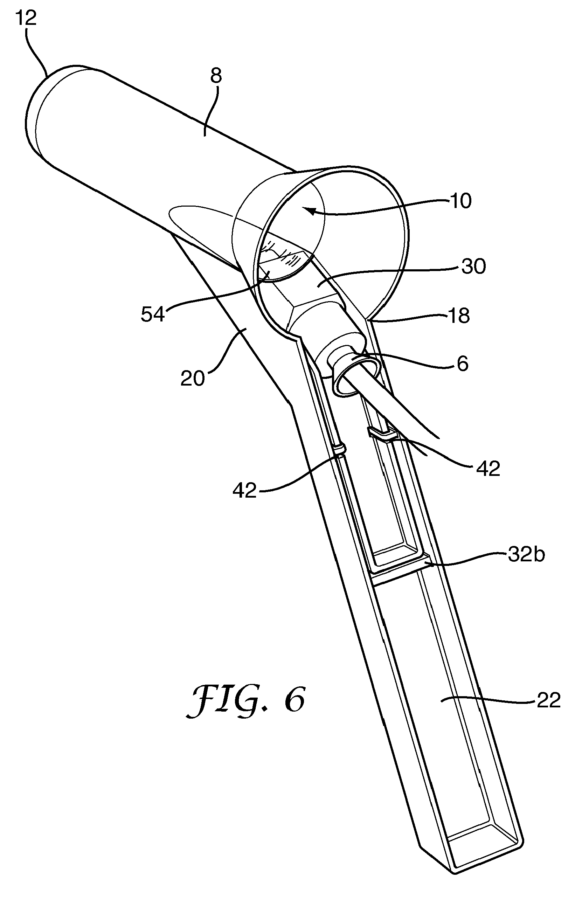

To shield the end 44 of the light source 6 from this foreign matter, Figures 3 to 6 show an embodiment of the present invention in which a translucent window pane 54 is provided between the proctoscope body 8 and the connecting portion 20.

The translucent window 54 sits within the slot 28 and, in this example, extends from the front of the slot to the front of the flange 16. This window 54 acts to block the passage of fluid or other foreign matter and prevents contamination of the light source 6.

The window 54 may be formed by the wall of the proctoscope body 8. In this case, the region of the proctoscope body 8 with a constant diameter would be formed by a continuous cylinder, and the slot 28 would only be present in the flange 16 to accommodate the light source 6. However, it may be advantageous to form the window 54 from material that is thinner than the walls of the proctoscope 2 to maximise the amount of light travelling down the bore 10 of the proctoscope body 8. Therefore, it may be preferable to form the window 54 from a plate of thinner plastic material which is glued or attached in another suitable way to the top of the connecting portion 20 between the proctoscope body 8 and the end 44 of the light source 6. Furthermore, the embodiments in Figures 3 to 6 show the window 54 having the same or similar curvature to the cylindrical proctoscope body 8, however, the window 54 may be planar or another suitable shape as long as no part of the window 54 projects into the bore of the proctoscope body 8 to allow insertion of the obturator 4.

The window 54 may optionally be formed as a lens to direct light from the light source 6 in a preferred direction, or to diffuse or de-focus the light beam.

The window 54 is integrally formed and continuous with the walls of the proctoscope body 8. The window 54 provides a fluid tight seal between the bore of the proctoscope body 8 so that liquids and gases are unable to pass through or around the window 54 and contaminate the end 44 of the light source 6.

The window 54 significantly reduces the likelihood that the light source 6 is contaminated during use and means, therefore, that the light source 6 may be used repeatedly without requiring replacement or sterilization between uses.

## Claims

1. A proctoscope comprising a tubular proctoscope body having a bore and a handle integrally formed with the proctoscope body; wherein
the proctoscope body has a distal end with an opening, for insertion into the anus of a patient, and a mounting member for supporting a light source for directing light down the bore towards the distal end;
**characterised in that** a translucent window is provided, which is integral with the proctoscope body and provides a fluid tight seal between the bore and the light source such that, in use, a light source supported in the mounting member is shielded from foreign matter passing along the bore of the proctoscope body.

2. A proctoscope as claimed in claim 1, wherein the window is curved.

3. A proctoscope as claimed in claim 1 or claim 2, wherein the window is a lens.

4. A proctoscope as claimed in claim 3, wherein the lens is adapted to focus and direct light from the light source in a preferred direction.

5. A proctoscope as claimed in claim 3, wherein the lens is adapted to diffuse or de-focus light from the light source.

6. A proctoscope as claimed in any preceding claim, further comprising a handle; wherein the window is in a side wall of the proctoscope body and is integrally formed with the handle.
